# EUROPÄISCHE PATENTSCHRIFT

(11) **EP 2 945 575 B1**
(45) Veröffentlichungstag und Bekanntmachung des Hinweises auf die Patenterteilung: **14.03.2018**
(21) Anmeldenummer: 14700557.3
(22) Anmeldetag: 17.01.2014
(51) Int. Cl.: A61F 2/50, A61F 2/78

(54) **PROTHESENELEMENT UND VERFAHREN ZUM HERSTELLEN EINES PROTHESENELEMENTES**
PROSTHESIS ELEMENT AND METHOD FOR PRODUCING A PROSTHESIS ELEMENT
ÉLÉMENT DE PROTHÈSE ET PROCÉDÉ POUR LE FABRIQUER

(30) Priorität: 18.01.2013 DE 102013000770
(43) Veröffentlichungstag der Anmeldung: 25.11.2015
(73) Patentinhaber: Otto Bock HealthCare GmbH, 37115 Duderstadt (DE)
(72) Erfinder: SAWATZKI, Steffen, 99759 Sollstedt (DE); SCHNEEGANS, Markus, 37434 Rollshausen (DE); ZARLING, Sven, 37115 Duderstadt (DE); NIEDERSTRASSER, Sandra, 37115 Duderstadt (DE); KRUSE, Andreas, 37339 Worbis (DE); SCHLESIGER, Ilka, 07745 Jena (DE); STOHR, Martin, 07745 Jena (DE); HAASE, Dirk, 07743 Jena (DE); GLÄSKER, Esther, 65187 Wiesbaden (DE)
(74) Vertreter: Stornebel, Kai
(86) Internationale Anmeldenummer: PCT/EP2014/000118
(87) Internationale Veröffentlichungsnummer: WO 2014/111263

(56) Entgegenhaltungen:
- WO-A1-00/51537
- WO-A1-2006/073353
- WO-A1-2013/079159
- US-A- 1 305 721
- US-A- 5 376 127
- US-A1- 2007 162 154

## Beschreibung

Die Erfindung betrifft ein Prothesenelement zur Verkleidung einer Prothese oder zur Ausbildung einer Prothesenkomponente sowie ein Verfahren zum Herstellen eines Prothesenelementes aus einem flächigen Grundmaterial.

Prothesen ersetzen fehlende Gliedmaßen in funktioneller und kosmetischer Hinsicht. Sie bestehen in der Regel aus mehreren Elementen, die miteinander zusammenwirken, um die an sie gestellten Anforderungen zu erfüllen. Neben einer Einrichtung zur Anbindung an den Prothesennutzer, beispielsweise in Gestalt eines Schaftes, in den ein verbliebener Teil einer Gliedmaße eingeführt wird, sind beispielsweise Gelenkeinrichtungen oder distal angeordnete Endelemente vorgesehen. Bei einer prothetischen Versorgung eines Patienten, der über einen Oberschenkelstumpf verfügt, sind die üblichen Komponenten ein Oberschenkelschaft, ein Prothesenkniegelenk, ein Unterschenkelrohr sowie ein Prothesenfuß. Bei Patienten, die über einen Oberarmstumpf verfügen, sind ein Oberarmschaft, ein Ellenbogengelenk, ein Unterarmrohr sowie eine Prothesenhand mit einem Prothesenhandgelenk vorgesehen. Diese Komponenten sind in der Regel nach funktionellen Gesichtspunkten gestaltet und weisen keine Form auf, die der natürlichen Gestalt angenähert ist. Insbesondere die Gestaltung der Gelenkeinrichtungen sowie der Verbindungen dazwischen, nämlich das Unterschenkelrohr und das Unterarmrohr, sind als reine Funktionselemente in Rohrform, was unter ästhetischen Gesichtspunkten unbefriedigend ist.

Die DE 299 579 B betrifft ein Kunstbein, das mittels einer Hülse am Oberschenkelstumpf befestigt wird. Ein Innenskelett ist vorhanden, das die Hülse über ein Prothesenkniegelenk mit einem gelenkig gelagerten Prothesenfuß verbindet. Eine elastische Hülle ist um den Unterschenkel herum angeordnet.

Die DE 622 314 B betrifft ein Kunstbein für Oberschenkelamputierte mit einer den Oberschenkelstumpf aufnehmenden Hülse und einem mittels Gabel daran angelenkten Unterschenkel aus zwei ineinandergleitenden Rohren. Um die mechanischen Einrichtungen ist eine nicht näher bezeichnete Kosmetik angeordnet.

Die US 3,545,009 A betrifft einen Oberschenkelschaft, der in seinem Umfang einstellbar ist. Der Schaft kann aus Holz oder Kunststoff oder einem faserverstärktem Kunststoff ausgebildet sein und weist Schlitze auf, so dass über einen Kniehebelverschluss der Umfang variiert werden kann.

Die DE 285 917 B1 betrifft ein künstliches Kniegelenk mit anschließender Unterschenkelhülse mit einem das Knie umschließenden Lederstück, das teilweise in Falten gelegt ist. Das Lederstück ist mit der Unterschenkelhülse verbunden, die aus zwei ineinander geschobenen, mit Zahnstangen versehenden Hülsen besteht. Die Zahnstangen sind federnd zueinander gelagert, so dass sie die Hülsen in der richtigen Gebrauchslage zueinander halten. Die Hülse besteht aus einem Innenstück, das aus einer Anzahl parallel nebeneinander liegender Stahlstreifen, die in einem Gewebe oder sonstigen Material eingebettet sind, besteht. Über die Innenhülse ist eine dünnwandige Stahlhülse angeordnet, die eine einer Zahnstange gegenüberliegende Verzahnung trägt. Die Stahlhülse ist von einer Holzhülse bedeckt, die vorteilhafterweise aus kreuzweise übereinander gelegtem Furnier besteht.

Die US 5, 376, 127 A betrifft eine Prothesenverkleidung und ein Verfahren zu dessen Herstellung aus einem thermoplastischen, zellulären Polyethylenflachmaterial, das zu einer hohlen, endseitig offenen und im wesentlichen konischen Gestalt geformt wird. Durch zunächst ausreichendes Erwärmen und anschließendes Aufbringen von Druck wird die konische Form in einer Form in die an eine natürliche Gliedmaße angenäherte Gestalt gebracht. Einander gegenüberliegende Seitenkanten werden ggf. überlappend aneinander durch Nähen oder Kleben befestigt.

Die US 2007/162154 A1 betrifft eine Prothesenverkleidung mit einem hohlen, flexiblen Grundkörper, der im Wesentlichen konisch geformt ist, um eine Prothesenkomponente aufzunehmen. Eine obere Öffnung dient zum Durchlass einer Gelenkeinrichtung, eine untere Öffnung lässt eine distale Komponente hervortreten, wobei die Verkleidung eine Schutzfunktion wahrnimmt. Über eine Naht können rückseitige Kanten miteinander verbunden werden.

Die WO 00/51537 betrifft eine orthetische oder prothetische Manschette mit zumindest zwei Textilelemente mit unterschiedlichen Elastizitäten, wobei die Richtung einer erhöhten Steifigkeit des einen Elements axial entlang einer Manschettenachse verläuft und die Richtung der erhöhten Steifigkeit des zweiten Elements transversal zu dieser Achse verläuft.

Aufgabe der vorliegenden Erfindung ist es, ein Prothesenelement zur Verkleidung einer Prothese oder zur Ausbildung einer Prothesenkomponente sowie ein Verfahren zur Herstellung eines Prothesenelementes bereitzustellen, mit denen eine kostengünstige Herstellung und eine einfache Anpassbarkeit an den jeweiligen Prothesennutzer möglich sind.

Erfindungsgemäß wird diese Aufgabe durch ein Prothesenelement mit den Merkmalen des Hauptanspruches und ein Verfahren mit den Merkmalen des nebengeordneten Anspruchs gelöst. Vorteilhafte Ausgestaltungen und Weiterbildungen der Erfindung sind in den jeweiligen Unteransprüchen aufgeführt.

Das Prothesenelement zur Verkleidung einer Prothese oder zur Ausbildung einer Prothesenkomponente besteht aus zumindest einem Zuschnitt aus einem flächigen Grundmaterial, der unter Ausbildung eines Hohlkörpers gebogen ist, wobei im gebogenen Zustand einander gegenüberliegende Ränder miteinander verbunden sind und an dem Grundmaterial zumindest ein Versteifungselement zur Erhöhung der Eigenstabilität des Prothesenelementes angeordnet ist, wobei an dem Zuschnitt oder den Zuschnitten Formschlusselemente, über die die Ränder aneinander festlegbar sind, oder Befestigungselemente zur formschlüssigen Festlegung des Prothesenelementes an einer Prothesenkomponente an dem Grundmaterial angeordnet sind. Während gegenwärtig kosmetische Verkleidungen für Prothesen der unteren Extremität aus einem vorgeformten Schaum von einem Orthopädietechniker angefertigt werden müssen, die zur Anpassung an die gewünschte anatomische Kontur überschüssiges Material abfräsen müssen, sieht das erfindungsgemäße Prothesenelement die Herstellung einer dreidimensionalen kosmetischen Verkleidung einer Prothese oder einer dreidimensionalen Prothesenkomponente aus einem flächigen Grundmaterial vor, das zugeschnitten wird. Der Zuschnitt aus diesem Material wird dann unter Ausbildung eines Hohlraumes zu einem dreidimensionalen Prothesenelement geformt und dauerhaft an den einander gegenüberliegenden Rändern verbunden. Als einander gegenüberliegende Ränder werden diejenigen Ränder des Zuschnittes angesehen, die im gebogenen Zustand einander gegenüberliegen, so dass die gewünschte Form und Kontur des Prothesenelementes fixiert wird. Neben einer rein kosmetischen Anwendung ist es möglich, dass das Prothesenelement auch funktionale Aufgaben wahrnimmt, beispielsweise Kräfte überträgt und somit Funktionskomponenten einer Prothese ausbildet und ersetzt, beispielsweise ein Unterschenkelrohr auf ein Unterarmrohr. Das Prothesenelement ist insbesondere für eine Erstversorgung geeignet.

An dem Grundkörper ist das Versteifungselement angeordnet, beispielsweise angeklebt, angeschweißt, angespritzt oder angeformt oder auf eine andere Art und Weise befestigt, um einerseits eine Optik zu erzeugen, die der natürlichen Gliedmaße, z.B. dem Knöchelbereich oder distalen Unterschenkelbereich, entspricht und andererseits eine funktionale Abdeckung der mechanischen Komponenten der Protheseneinrichtung bereitzustellen. Das Versteifungselement ermöglicht es, dass das Prothesenelement eine ausreichende Formstabilität beim Bewegen der Protheseneinrichtung aufweist, ohne dass die Beweglichkeit zu sehr eingeschränkt wird. Ebenso ist eine ausreichende Verformbarkeit durch den flexiblen Grundkörper gewährleistet, so dass eine natürliche Anmutung weiterhin gegeben ist. Über das Versteifungselement ist es möglich, die Funktionalität des Prothesenelementes an die gewünschten Eigenschaften anzupassen, nämlich dass eine ausreichend große Stabilität vorhanden ist, um ein Herunterrutschen oder ein Abrutschen zu verhindern oder grundsätzlich eine ausreichende Steifigkeit bereitzustellen, ohne dass eine Flexibilität verloren geht, die benötigt wird, um ein Falten oder Umschlagen des Grundkörpers zu vermeiden, wie es bei einem Einknicken eines starren Materials, etwa dem eines Gummistiefels, der Fall wäre.

Das Grundmaterial kann aus einem Schaumstoff, einem 3D-Gewirk oder einem Kunststoff bestehen, ebenfalls ist es möglich, dass ein Metall zum Einsatz kommt, um aus dem Grundmaterial einen entsprechenden Zuschnitt zu erzeugen. Als Kunststoff kann auch ein faserverstärkter Kunststoff verwendet werden, wenn eine dauerhafte und/oder eine kraftübertragende Ausgestaltung des Prothesenelementes gewünscht wird. Bei einer Ausgestaltung des Grundmaterials aus einem Schaumstoff oder einem 3D-Gewirk ist eine besonders einfache Herstellung des Prothesenelementes gewährleistet, ebenso ist das Prothesenelement besonders leicht und kostengünstig zu fertigen.

Neben einer einstückigen Ausgestaltung des Zuschnittes ist es möglich, dass mehrere Zuschnitte aus dem Grundmaterial hergestellt und die im Endzustand des Hohlkörpers einander gegenüberliegenden Ränder auch unterschiedlicher Zuschnitte miteinander verbunden sind.

Der Zuschnitt oder die Zuschnitte können an den jeweils korrespondierenden, einander gegenüberliegen Rändern miteinander verklebt und/oder verschweißt werden, um eine dauerhafte Verbindung der einander gegenüberliegenden, korrespondierenden Ränder miteinander zu erlangen.

Neben einer möglichen stoffschlüssigen und kraftschlüssigen Verbindung ist es erfindungsgemäß vorgesehen, dass an dem Zuschnitt oder den Zuschnitten Formschlusselemente angeordnet oder ausgebildet sind, über die die jeweiligen Ränder aneinander festlegbar sind, so dass sich im verbundenen Zustand der Hohlkörper ausbildet. Die Formschlusselemente können an einer separaten Komponente ausgebildet sein und einer Verlagerung der Ränder zueinander in zumindest einer Richtung verhindern oder behindern. Ebenso können die Formschlusselemente Teil des Zuschnittes sein, beispielsweise in Gestalt von korrespondierenden Ausschnitten oder Ausnehmungen.

Eine Ausgestaltung der Erfindung sieht vor, dass der Zuschnitt einen proximalen und einen distalen Abschlussrand aufweist, wobei die Bezeichnung proximal und distal in Bezug auf den fertiggestellten, angelegten Zustand des Prothesenelementes bezogen ist. Von dem distalen Abschlussrand erstreckt sich zumindest ein Ausschnitt in proximaler Richtung, so dass Umfangsveränderungen am distalen Abschlussrand verwirklicht werden können. Ebenso ist es ergänzend oder alternativ vorgesehen, dass sich vom proximalen Abschlussrand zumindest ein Ausschnitt in distaler Richtung erstreckt, um dort eine gewölbte Form realisieren zu können. Dies ist insbesondere bei Prothesenelementen, die als Unterschenkelkosmetik verwendet werden, vorteilhaft, um die Einschnürungen und Rundungen im Knöchelbereich und im Kniebereich realistisch abbilden zu können. Die jeweiligen Ausschnitte von den Rändern in das Innere des Zuschnittes verjüngen sich vorteilhafterweise von dem jeweiligen Abschlussrand weg, so dass sich nach der Verbindung der Ränder des Ausschnittes bei der Ausbildung des Hohlkörpers eine Einwölbung in Richtung auf das Innere des Hohlkörpers hin ergibt.

An einem distalen oder proximalen Abschlussrand können Einrichtungen zur Festlegung des Prothesenelementes an weiteren Prothesenkomponenten angeordnet oder ausgebildet sein, beispielsweise können an dem proximalen Ende Einrichtungen zur Festlegung an einem Aufnahmeschaft für einen Stumpf ausgebildet sein, während am distalen Ende Aufnahmeeinrichtungen für weitere Prothesenkomponenten vorhanden sind, beispielsweise Prothesenhände oder Prothesenfüße.

Der aus dem Zuschnitt gebildete Hohlkörper ist im Querschnitt vorzugsweise geschlossen ausgebildet, um keine unnatürlich erscheinenden Öffnungen innerhalb des Hohlkörpers entstehen zu lassen.

Auf dem Grundmaterial können mehrere Schnittlinien für Zuschnitte unterschiedlicher Größen aufgebracht sein, so dass, ähnlich einem Schnittmuster, für unterschiedliche Größen ein Orthopädietechniker nach einer ersten Vermessung des Patienten bereits eine Auswahl des zu verwendenden Zuschnittes treffen kann. Eine individuelle Anpassung an den jeweiligen Patienten, insbesondere zur Angleichung an eine unversorgte Gliedmaße, ist immer noch möglich.

Das Prothesenelement kann als Prothesenkosmetik oder als ein lasttragendes Strukturbauteil ausgebildet sein, beispielsweise als Verbindungseinrichtung zwisehen einem Prothesenfuß und einem Prothesenkniegelenk.

Das Verfahren zum Herstellen eines Prothesenelementes am flächigen Grundmaterial sieht die Schritte des Ausschneidens eines Zuschnittes aus dem flächigen Grundmaterial vor. Das Ausschneiden kann auf unterschiedliche Arten ausgeführt werden, beispielsweise über Scheren, Laser oder mittels Wasserstrahlschneiden. Anschließend wird der Zuschnitt unter Ausbildung eines Hohlkörpers gebogen, so dass Ränder des Zuschnittes einander gegenüberliegen. Die nach dem Biegen des Zuschnittes einander gegenüberliegenden Ränder werden dann miteinander über Formschlusselemente, die an dem Zuschnitt angeordnet sind, verbunden, so dass ein im Wesentlichen geschlossener Hohlkörper üblicherweise mit Öffnungen für das Einführen von Prothesenkomponenten an den distalen und proximalen Enden ausgebildet wird. An dem Grundmaterial wird zumindest ein Versteifungselement zur Erhöhung der Eigenstabilität des Prothesenelementes angebracht.

Eine Weiterbildung der Erfindung sieht vor, dass der Zuschnitt um die Prothese herum gebogen und anschließend die Ränder miteinander verbunden werden. Dadurch ist es möglich, bei einer Ausgestaltung des Prothesenelementes als eine Kosmetik die funktionalen Komponenten der Prothese zunächst aufeinander abzustimmen und anschließend an der fertig aufgebauten Prothese das Prothesenelement anzulegen.

Der Zuschnitt kann entlang vorher auf dem Grundmaterial aufgebrachter Schnittlinien ausgeschnitten werden, so dass statt einer freihändigen Ausgestaltung des Zuschnittes der Orthopädiemechaniker eine vorgefertigte Form als Leitlinie enthält, an der er sich orientieren kann. Insbesondere bei dem Vorhandensein von Aufnahmen für Anschlusselemente zu anderen Prothesenkomponenten sind die Schnittlinien vorteilhaft, so dass die exakte Lage der jeweiligen Ausschnitte feststeht und die Herstellung des Prothesenelementes wesentlich vereinfacht wird.

Neben einer permanenten Verbindung der korrespondierenden, einander gegenüberliegenden Ränder ist es möglich, dass diese auch reversibel miteinander verbunden werden, so dass bei einer nur vorläufigen Versorgung mit dem erfindungsgemäßen Prothesenelement ein einfaches Entfernen möglich ist. Die reversible Verbindung kann durch ein oder mehrere separate oder im Zuschnitt ausgebildet Formschlusselemente erfolgen. Alternativ dazu werden die Ränder vorzugsweise verklebt oder verschweißt, um eine dauerhafte Zuordnung der Ränder zueinander zu gewährleisten.

Es können mehrere Zuschnitte unter Ausbildung des Hohlkörpers miteinander verbunden werden, falls die Form des Prothesenelementes zu komplex ist, um die gewünschte Hohlkörperkontur zu erreichen.

Das Prothesenelement kann für eine Protheseneinrichtung mit einem Prothesenfuß und einem Oberschenkel zur Überbrückung eines zwischen dem Prothesenfuß und dem Oberschenkel vorhandenen Freiraumes eingesetzt werden und aus dem Zuschnitt des flexiblen Grundmaterials gebildet sein. Der Zuschnitt bildet einen Grundkörper, der einen Hohlraum zur Aufnahme der Protheseneinrichtung umfasst, wobei an dem Grundkörper zumindest ein Versteifungselement zur Erhöhung der Eigenstabilität des Prothesenelementes angeordnet ist. Das Prothesenelement kann als Manschette, beispielsweise Knöchelmanschette ausgebildet sein und zur Überbrückung eines Freiraumes oder Überganges zwischen zwei Prothesenkomponenten, beispielsweise dem oberen Rand oder dem proximalen Ende des Prothesenfußes und einem Unterschenkelteil, beispielsweise einem Gehäuse einer Dämpfer- und Steuereinrichtung oder einer anderen, das Volumen eines natürlichen Unterschenkels nachbildenden und/oder die Funktion eines Unterschenkelrohrs ausübenden Komponente eingesetzt werden. Andere Einsatzgebiete sind ebenfalls möglich, beispielsweise Unterschenkelkosmetiken, Abdeckungen an oberen oder unteren Extremitäten und Last abtragenden Einrichtungen an Prothesen. Der Grundkörper aus dem Zuschnitt bildet im angelegten Zustand einen Hohlraum aus, in dem die Protheseneinrichtung, z.B. ein Teil des Unterschenkelteils, angeordnet ist. Der Grundkörper kann somit einen distalen Abschnitt des Unterschenkelteils im Bereich des Überganges zum Prothesenfuß umgeben, bei anderen Anwendungsgebieten werden andere Freiräume überbrückt.

Das Versteifungselement besteht dabei aus einem Material, das einen gegenüber dem Grundmaterial oder dem Material des Grundkörpers größeren Verformungswiderstand aufweist, so dass gezielt an denjenigen Stellen, an denen das Versteifungselement angeordnet ist, die gewünschte Formstabilität und der gewünschte Verformungswiderstand erzielt bzw. erhöht werden kann. Ebenso ist es möglich, durch das Versteifungselement an besonders empfindlichen Stellen der Prothese, z.B. im Übergang zwischen einem Prothesenfuß und einem Unterschenkelteil oder im Bereich eines Ellenbogengelenkes, eine verbesserte mechanische Schutzwirkung bereitzustellen.

Das Grundmaterial kann aus einem Textil und/oder einem Schaumstoff hergestellt sein bzw. ein Textil und/oder einen Schaumstoff aufweisen und weitere Materialien umfassen, sofern dies notwendig ist. Textile und Schaumstoffe haben den Vorteil einer einfachen Herstellbarkeit, Verarbeitbarkeit und eines leichten Gewichtes bei ausreichender Flexibilität und Verformbarkeit. Die Textilien können als Gewebe, Gewirke, Gestricke und als Abstandsgewirke ausgebildet sein, als Schaumstoffe sind sowohl offenporige als auch geschlossenporige Schaumstoffe vorgesehen. Das Grundmaterial kann eine Beschichtung aufweisen, die die Wasserundurchlässigkeit des Prothesenelementes erhöht oder herstellt.

Zum formschlüssigen Festlegen des Prothesenelementes an einer Prothesenkomponente, z.B. an einem Prothesenfuß, einem Oberschenkelschaft, einem Unterschenkelteil oder einem Unterarmschaft, können erfindungsgemäß Befestigungselemente an dem Grundmaterial oder dem Versteifungselement angeordnet sein, so dass eine Relativbewegung zwischen dem Prothesenelement und der weiteren Prothesenkomponente, an der das Prothesenelement festgelegt wird, während der üblichen Benutzung der Prothese nicht erfolgen kann. Dadurch ist es möglich, das Prothesenelement bündig oder nahezu bündig mit dem jeweiligen Anschlusselement oder Anschlussbereich der Prothesenkomponente auszubilden bzw. zu orientieren. Die Befestigungselemente zum formschlüssigen Festlegen bewirken zudem, dass sich die Kontur des Prothesenelementes an die Kontur der Prothesenkomponente anpasst, so dass es möglich ist, unterschiedliche Formen oder Größen von Prothesenkomponenten mit ein und demselben Prothesenelement zu kombinieren.

Eine Weiterbildung der Erfindung sieht vor, dass das Versteifungselement als ein Rahmen ausgebildet ist, der den Zuschnitt oder das Grundmaterial, das den Grundkörper ausbildet, umgibt. Der Rahmen kann umlaufend um den gesamten Umfang des Grundkörpers herum angeordnet sein, so dass eine ausreichende Formstabilität zur Montage des Prothesenelementes beispielsweise an einem Prothesenfuß oder an einem Unterschenkelteil gegeben ist. Durch den Rahmen wird der Hohlraum, der von dem Grundkörper umgeben wird, definiert, so dass der Grundkörper selbst aus einem biegeschlaffen Material ausgebildet sein kann, das durch das Versteifungselement in der gewünschten Form gehalten wird.

In dem Versteifungselement kann zumindest eine Scharniereinrichtung zur Erleichterung einer Flexion um ein Gelenk angeordnet sein. Diese Scharniereinrichtung erleichtert die Relativbewegung zwischen zwei Prothesenkomponenten, z.B. dem Prothesenfuß und dem Unterschenkelteil, und verhindert, dass Geräusche beim Einbeugen durch ein Auffalten des Prothesenelementes entstehen. Weiterhin werden die Auszugskräfte verringert, die bei einer Flexion um ein Gelenk auftreten. Durch die Reduzierung der Auszugskräfte wird gewährleistet, dass das Prothesenelement in der vorgesehenen Position relativ zu den Prothesenkomponenten verbleibt.

Das Prothesenelement ist vorteilhafterweise symmetrisch ausgebildet, wobei die Symmetrie vorzugsweise zur Sagittalebene besteht, so dass sie sowohl für die Anwendung an einer linken als auch an einer rechten Protheseneinrichtung geeignet ist.

Das Versteifungselement kann den distalen und/oder proximalen Abschluss des Prothesenelementes bilden, wobei zumindest Teile des Versteifungselementes den distalen und/oder proximalen Abschluss gestalten. Dadurch ist es möglich, dass insbesondere Befestigungselemente, die naturgemäß eine größere Festigkeit als das biegeschlaffe oder flexible Material des Grundkörpers aufweisen müssen, leichter hergestellt und angeformt werden können. Bei einer separaten Ausgestaltung der Befestigungselemente ist es möglich, durch die Anordnung am distalen und/oder proximalen Ende des Prothesenelementes eine einfache Zuordnung der Befestigungselemente sowohl zu dem Prothesenelement als auch zu der jeweiligen Prothesenkomponente zu erreichen. Die Befestigungselemente können dabei formschlüssig an einem Formschlusselement an dem Prothesenelement festgelegt werden, wobei das Formschlusselement an dem Versteifungselement ausgebildet oder befestigt ist.

Auf der Innenseite, insbesondere im proximalen Bereich des Grundmaterials und damit des Grundkörpers kann eine reibungsvermindernde Beschichtung angeordnet sein, ebenso können reibungsvermindernde Elemente auf der Innenseite des Grundmaterials angeordnet sein, beispielsweise angeklebt, angespritzt, angeschweißt oder auf andere Art und Weise an dem Grundmaterial befestigt sein. Durch eine reibungsvermindernde Beschichtung ist es möglich, eine Relativbewegung zwischen dem Prothesenelement und der Prothesenkomponente zu ermöglichen, ohne dass hohe Auszugskräfte auf das Prothesenelement, z.B. Knöchelmanschette, wirken, so dass eine feste Zuordnung zu der Prothesenkomponente, z.B. zwischen dem distalen Ende der Knöchelmanschette und dem proximalen Ende des Prothesenfußes, erhalten bleibt.

Das Versteifungselement kann einen geschlossenen Querschnitt aufweisen, um die Formstabilität weiter zu erhöhen. Die Form ist in der Regel oval oder einem Oval angenähert. Das Versteifungselement kann sich über einen größeren Höhenbereich in Proximal-Distal-Erstreckung erstrecken, wobei Ausschnitte in dem Versteifungselement, insbesondere in anteriorer und/oder posteriorer Orientierung, vorgesehen sein können. Durch diese Ausschnitte ist es möglich, eine Verformung des Grundmaterials zu ermöglichen. Dazu ist es vorgesehen, dass das Versteifungselement auf dem Grundmaterial bzw. Grundkörper aufgebracht ist, so dass die gegebenenfalls vorgesehenen Ausschnitte in dem Versteifungselement von dem Grundmaterial abgedeckt sind Das Prothesenelement ist somit im angelegten Zustand vorzugsweise geschlossen, so dass keine Feuchtigkeit und/oder Schmutz eindringen kann.

Ebenso kann es vorgesehen sein, dass der Grundkörper einen offenen Querschnitt aufweist und das Versteifungselement auch oder nur entlang der Öffnung angeordnet ist und den Querschnitt schließt, also die einander gegenüberliegenden Kanten des Zuschnittes des Grundkörpers aufnimmt, fixiert und insgesamt einen geschlossenen Querschnitt für das Prothesenelement bereitstellt.

Nachfolgend werden Ausführungsbeispiele der Erfindung anhand der beigefügten Figuren näher erläutert. Es zeigen:
- Figur 1a: - ein Grundmaterial mit einem teilausgeschnittenen Zuschnitt;
- Figur 1b: - einen Zuschnitt gemäß Figur 1a im teilweise gebogenen Zustand;
- Figur 2: - einen Zuschnitt für eine Oberschenkelkosmetik;
- Figur 3: - einen Zuschnitt für einen Fußanschluss;
- Figur 4: - fertig ausgebildete Prothesenelemente;
- Figur 5: - eine Variante der Figur 1b mit einem Formschlusselement;
- Figur 6: - eine Detailschnittansicht einer Stoßstelle;
- Figur 7: - eine Variante der Figur 6;
- Figur 8: - eine Seitenansicht einer Protheseneinrichtung mit Knöchelmanschette;
- Figur 9: - eine Ansicht einer Knöchelmanschette von schräg hinten;
- Figur 10: - eine Einzeldarstellung einer Variante einer Knöchelmanschette;
- Figur 11: - eine Seitenansicht gemäß Figur 10;
- Figur 12: - eine Rückansicht gemäß Figur 10;
- Figur 13: - eine Frontalansicht gemäß Figur 10;
- Figur 14: - eine Einzelteildarstellung einer Variante;
- Figur 15: - eine Detailansicht der Variante gemäß Figur 14;
- Figur 16: - eine Teilschnittdarstellung der Variante gemäß Figur 14 in Schrägdraufsicht; sowie
- Figur 17: - eine Variante der Erfindung in Seitenansicht.

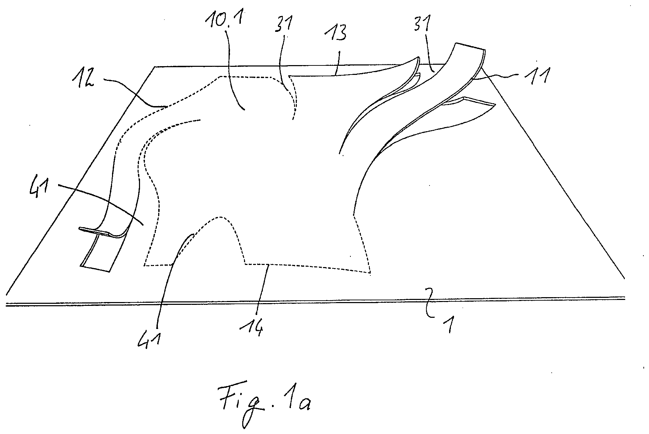

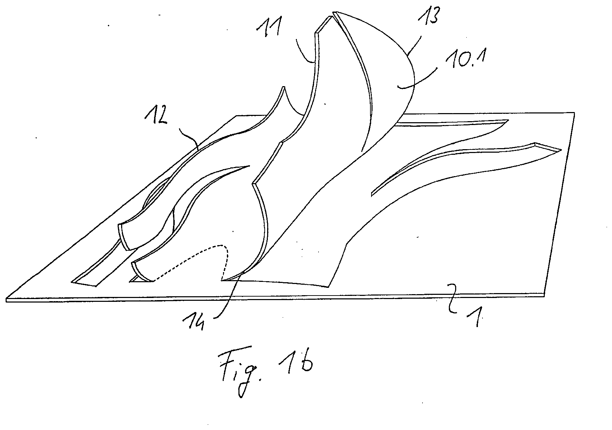

In der Figur 1 a ist ein Grundmaterial 1 dargestellt, das als Kunststoff, Schaumstoff, 3D-Gewirke oder auch als Metalltafel ausgebildet sein kann. Aus dem Grundmaterial 1, das flach auf einer Unterlage liegt, ist ein Zuschnitt 10.1 bereits teilweise ausgeschnitten, die Kontur des Zuschnittteiles, die noch nicht ausgeschnitten ist, ist durch eine Strichlinie gekennzeichnet. Der Zuschnitt 10.1 ist für die Ausbildung einer Unterschenkelkosmetik ausgebildet und weist einen proximalen Abschlussrand 13 und einen distalen Abschlussrand 14 auf. Der distale Abschlussrand 14 ist im fertiggestellten Zustand einem Prothesenfuß zugeordnet, während der proximale Abschlussrand 13 einem Prothesenkniegelenk zugeordnet ist. Von den Abschlussrändern 13, 14 erstrecken sich mehrere Ausschnitte 31, 41 in Richtung auf das Innere des Zuschnittes 10.1. Von dem proximalen Abschlussrand 13 erstrecken sich die Ausschnitte 31 im Wesentlichen in distaler Richtung, während die Ausschnitte 41 vom distalen Rand sich im Wesentlichen in proximaler Richtung erstrecken. Über die Form der Ausschnitte 31, 41, die vom jeweiligen Rand 13, 14 weg sich verjüngend darstellt, wird die Form des später daraus gebildeten Hohlkörpers definiert.

Neben den distalen und proximalen Abschlussrändern 13, 14 sind laterale Ränder 11, 12 vorgesehen, die korrespondierend zueinander ausgebildet sind, so dass sie nach dem Biegen des Zuschnittes 10.1 um eine Längsachse, die im Wesentlichen von proximal nach distal verläuft, einander gegenüberliegen und entlang der Längserstreckung des Prothesenelementes verlaufen.

In der Figur 1b ist der Zuschnitt 10.1 in einem weiter ausgeschnittenen und gebogenen Zustand dargestellt. Es ist zu erkennen, dass die Ränder der Ausnehmungen 31, 41 bereits aufeinander zu bewegt sind. Dadurch ergibt sich nach dem Verbinden der korrespondierenden Ränder, die einander im jeweiligen Ausschnitt 31, 41 gegenüberliegen, eine im Wesentlichen geschlossene Fläche des Zuschnittes 10.1. Wenn die seitlichen Ränder 11, 12 nach einem weiteren Biegen des Zuschnittes 10.1 aufeinander zu einander gegenüberliegen, werden auch diese miteinander verbunden, so dass sich ein im Wesentlichen geschlossener Querschnitt senkrecht zur Längserstreckung, also vom proximalen Abschlussrand 13 zum distalen Abschlussrand 14 hin, ergibt.

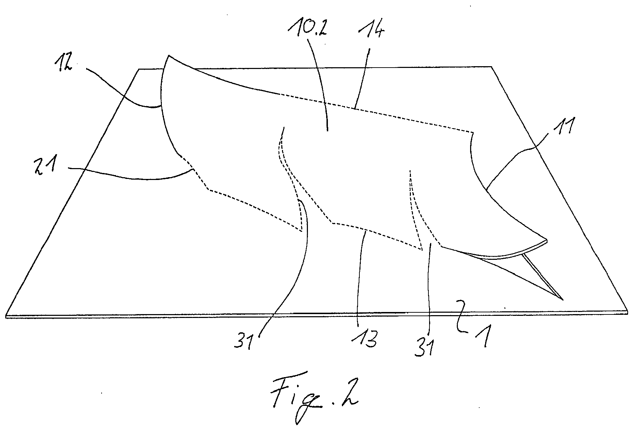

In der Figur 2 ist das Grundmaterial 1 und ein Zuschnitt 10.1 für ein Oberschenkelprothesenelement dargestellt, das insbesondere als Verkleidung oder auch als Schaftelement ausgebildet ist. Auch hier sind distale und proximale Abschlussränder 13, 14 vorhanden. Im dargestellten Ausführungsbeispiel sind nur vom proximalen Abschlussrand 13 ausgehende Abschnitte vorgesehen, um eine gewölbte, im Wesentlichen tonnenförmige Form des Prothesenelementes nach dem Fügen der seitlichen Ränder 11, 12 miteinander auszubilden. In der Figur 2 ist eine Schnittlinie 21 als Strichpunktlinie dargestellt, entlang derer der Zuschnitt 10.2 aus dem Grundmaterial 1 getrennt werden kann, vorzugsweise wird der Zuschnitt 10.2 herausgeschnitten, beispielsweise über einen Laserstrahl, einen Wasserstrahl, über einen Heizdraht, ein Messer oder eine Schere. Auch andere Trennverfahren sind möglich, um den Zuschnitt 10.2 aus dem Grundmaterial 1 herauszutrennen.

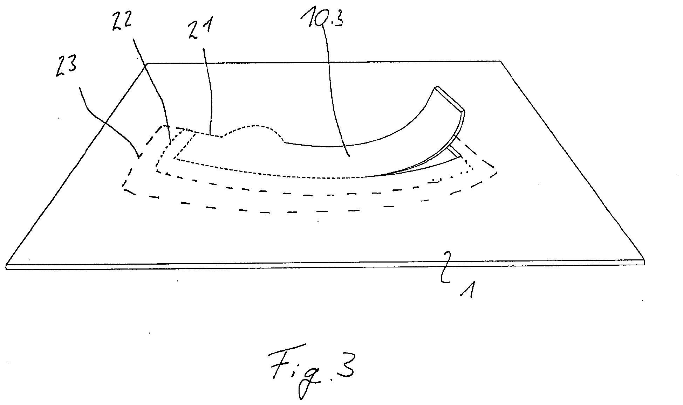

Die Figur 3 zeigt einen dritten Zuschnitt 10.3 in Gestalt eines Fußanschlusses. Auf dem Grundmaterial 10 sind mehrere Schnittlinien 21, 22, 23 für Zuschnitte 10.3 unterschiedlicher Größe vorgesehen, so dass ein Orthopädietechniker auf einfache Art und Weise den Zuschnitt für unterschiedliche Größen aus dem Grundmaterial 1 heraustrennen kann.

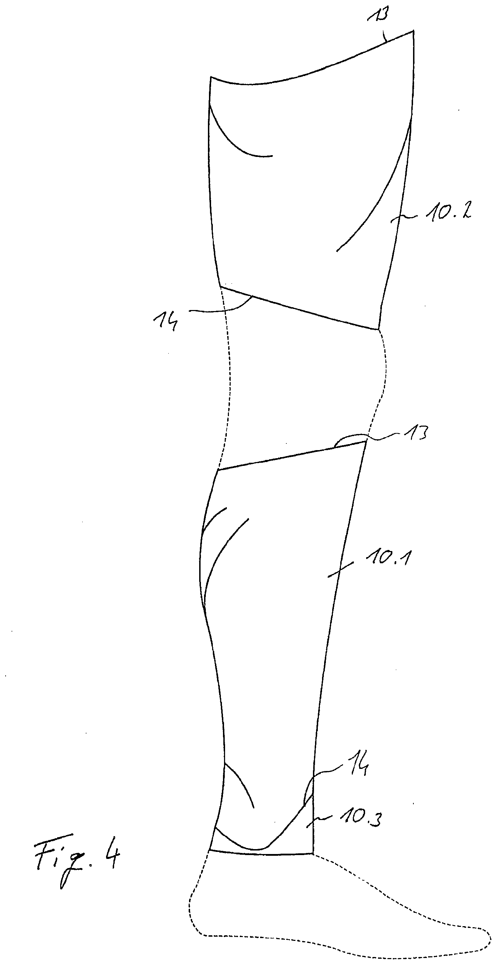

In der Figur 4 sind die drei als Zuschnitte in den Figuren 1 bis 3 dargestellten und erläuterten Prothesenelemente in fertiggestellter Form dargestellt. Die Oberschenkelverkleidung aus dem Zuschnitt 10.2 weist einen geschlossenen Querschnitt auf, die durch die Ausschnitte 31 gebildeten Ränder sind miteinander verbunden, so dass sich ein geschlossener Querschnitt ergibt. Über die sich verjüngenden und geschwungenen Ausschnitte in Richtung auf den ge-genüberliegenden Abschlussrand wird die sich zu den Abschlussrändern hin verjüngende, tonnenförmige Kontur erreicht.

Das mittlere Prothesenelement aus dem Zuschnitt 10.1 ist als Unterschenkelteil ausgebildet und weist eine Kontur auf, die der natürlichen Kontur eines Unterschenkels entspricht. An dem distalen Abschlussrand 14 ist der Fußanschluss auf dem Zuschnitt 10.3 angeordnet, vorzugsweise fest daran befestigt, beispielsweise durch Verschweißen oder Verkleben, so dass sich eine vollständige Unterschenkelkosmetik, die sich an den Prothesenfuß anschließt, ergibt. Grundsätzlich ist es auch möglich, bei einer entsprechenden Materialwahl, dass die aus den Zuschnitten 10.1, 10.2 und 10.3 hergestellten Prothesenelemente auch als lasttragende Prothesenelemente darstellen.

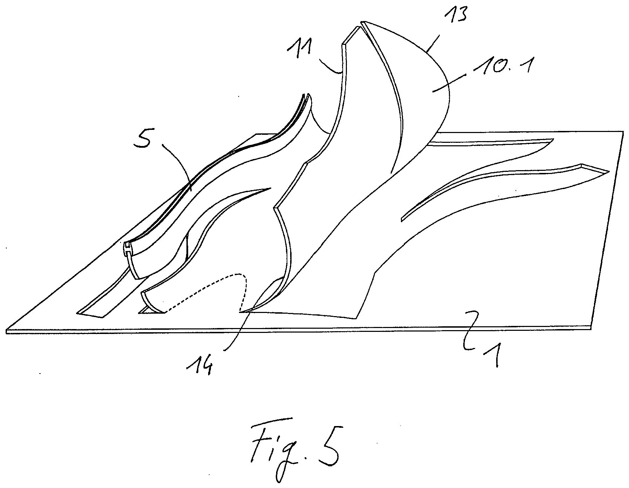

In der Figur 5 ist eine Variante der Figur 1b dargestellt, bei der an einem lateralen Rand 12 ein Formschlusselement 5 in Gestalt einer Profilleiste aufgebracht ist. Das Formschlusselement 5 erstreckt sich über die gesamte Länge des Randes 12 und weist eine Ausnehmung auf, in der der Zuschnitt 10.1 im Bereich des Randes 12 aufgenommen und festgelegt ist. Die Festlegung an dem Zuschnitt 10.1 kann klemmend oder formschlüssig erfolgen. Auf der dem in der Zeichnung linken Rand 12 gegenüberliegenden Seite des Zuschnittes 10.1 ist der noch nicht in das Formschlusselement 5 eingeführte Rand 11 zu erkennen, das Formschlusselement 5 weist auf der dem bereits eingeführten Rand 12 gegenüberliegenden Seite eine Ausnehmung auf, die korrespondierend zu der Ausnehmung zur Aufnahme des bereits eingeführten Randes 12 ausgebildet ist, so dass zum Montieren und Schließen des Zuschnittes 1 entlang der lateralen Ränder 11, 12 der rechte laterale Rand 11 lediglich in diese Ausnehmung eingeführt werden muss. Auch dort wird der Zuschnitt 10.1 klemmend oder formschlüssig gehalten.

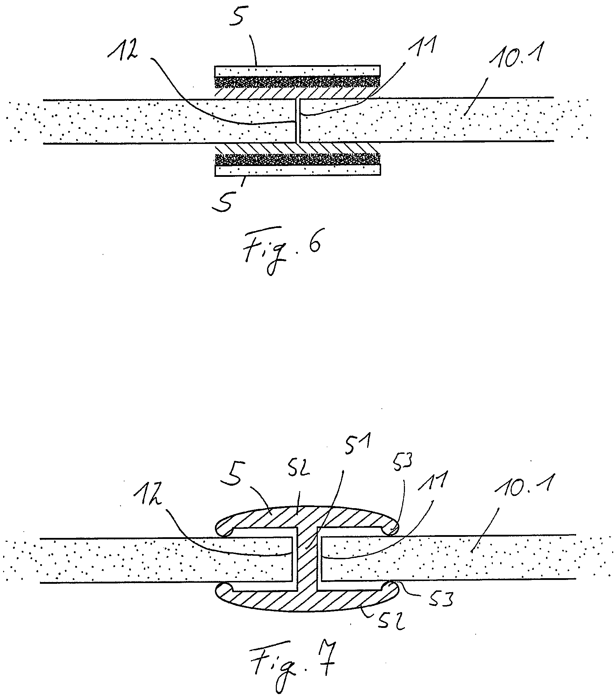

Die Figur 6 zeigt in einer Schnittdarstellung eine Variante der Befestigung und Zuordnung der lateralen Ränder 11, 12 des Zuschnittes 10.1 aneinander. Auf der Oberseite ebenso wie auf der Unterseite des Zuschnittes 10.1 sind die Stoßstelle zwischen den lateralen Rändern 11, 12 überbrückende Klettverschlüsse 5 angeordnet, über die eine formschlüssige Fixierung der Ränder 11, 12 zueinander erfolgen kann. An der Außenseite und/oder der Innenseite des Zuschnittes 10.1 sind vorteilhafterweise Flauschbereiche aufgebracht oder ausgebildet, so dass die Hakenbereiche eines Klettverschlusses 5 unmittelbar eingreifen können. Alternativ zu der Aufbringung von Flauschbereichen ist es möglich, dass die Oberfläche des Zuschnittmaterials einen Eingriff der Hakenbereiche der Klettverschlüsse 5 zulässt, so dass eine unmittelbare formschlüssige Verriegelung der Hakenbereiche der Klettverschlüsse 5 mit der Oberfläche des Zuschnittes 10.1 erfolgen kann. Zum Schließen des Zuschnittes 10.1 ist es dann lediglich erforderlich, Streifen von Klettverschlüssen bzw. Hakenbereichen von Klettverschlüssen 5 über die Stoßstelle der einander gegenüberliegenden lateralen Ränder 11, 12 zu legen und dort zu verankern. Die Klettverschlüsse können sich entlang der Längserstreckung der Stoßstelle erstrecken und über die gesamte Länge dimensioniert sein, alternativ kann die Orientierung der Klettverschlüsse senkrecht oder diagonal zu der Längserstreckung der Stoßstelle zwischen den beiden lateralen Rändern 11, 12 verlaufen.

Eine Variante der Festlegung der beiden Ränder 11, 12 aneinander und zueinander ist in der Figur 7 dargestellt, in der das Klemmprofil gemäß Figur 5 in einer Querschnittsdarstellung gezeigt ist. Das Formschlusselement 5 ist in der dargestellten Position als liegendes H ausgebildet und weist einen senkrechten Verbindungssteg 51 und zwei waagerecht verlaufende Ausleger 52 auf, die sich beiderseits des Verbindungssteges 51 erstrecken. An den Enden der Ausleger 52 können Vorsprünge 53 ausgebildet sein, die in einer Vorspannung auf das Zuschnittmaterial ausgebildet sind, um den Zuschnitt 10.1 sicher zwischen sich zu halten. Der Verbindungssteg 51 entspricht in seiner Länge im Wesentlichen der Materialstärke des Zuschnittmaterials, so dass die lateralen Ränder 11, 12 vollständig in die durch den Verbindungssteg 51 und die Ausleger 52 gebildeten Aufnahmeraum eingeführt werden können. Die Ausleger 52 verhindern eine Verlagerung der Ränder 11, 12 des Zuschnittes 10.1 parallel zu der Längserstreckung des Verbindungssteges 51, die Vorsprünge 53 eine Verlagerung der Ränder 11, 12 aufeinander zu oder voneinander weg. Sofern der Zuschnitt 10.1 nicht in das Formschlusselement 5 eingeführt ist, kann der Abstand zwischen den Vorsprüngen 53 kleiner als die Materialstärke des Zuschnittes 10.1 ausgebildet sein, die Ausleger 52 sind vorteilhafterweise elastisch ausgebildet und bewirken nach dem Einführen des Zuschnittes 10.1 in das Formschlusselement 5 eine Vorspannung und eine klemmende Haltung.

Zusätzlich oder alternativ zu dieser klemmenden Haltung ist es möglich, dass zu den Vorsprüngen 53 korrespondierende Ausnehmungen innerhalb des Zuschnittes 10.1 vorhanden sind, so dass zumindest über einen Teil der Gesamtlänge des Formschlusselementes 5 eine in Auszugsrichtung oder Einführrichtung des Zuschnittes 10.1 wirksame formschlüssige Verriegelung stattfinden kann. Die Ausleger 52 mit den in Richtung auf den Zuschnitt 10.1 gerichteten Vorsprüngen 53 können sich über die gesamte Länge des Formschlusselementes 5 erstrecken, alternativ dazu kann nur eine abschnittsweise Ausgestaltung des Formschlusselementes 5 mit den Auslegern 52 und den Vorsprüngen 53 vorgesehen sein.

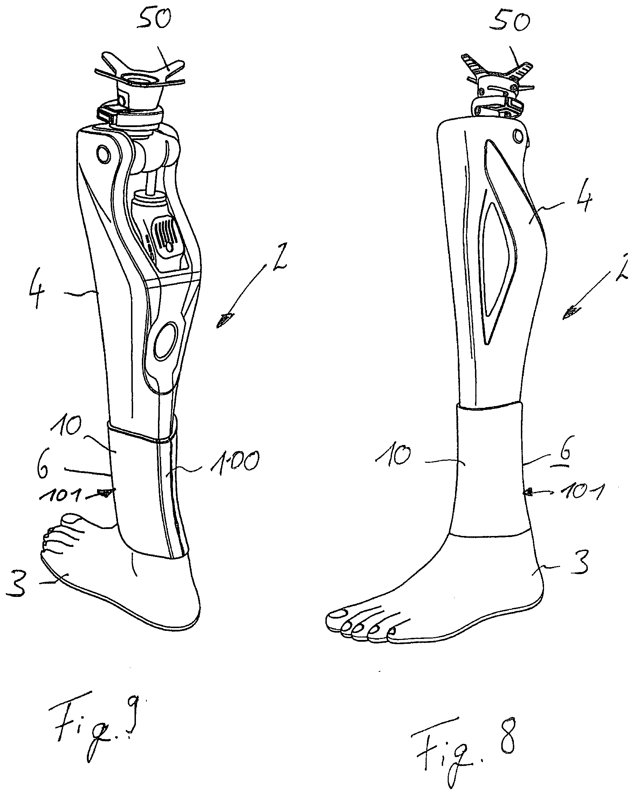

In der Figur 8 ist in einer Gesamtansicht eine Prothese oder Protheseneinrichtung 2 gezeigt, die einen Prothesenfuß 3, ein an dem proximalen Ende des Prothesenfußes 3 befestigten Unterschenkelteil 4 und eine am proximalen Ende des Unterschenkelteils 4 angeordneten Befestigungseinrichtung 50 aufweist. Die Befestigungseinrichtung 50 dient zur Montage der Protheseneinrichtung 2 an einem nicht dargestellten Oberschenkelschaft. Die Befestigungseinrichtung 50 ist gelenkig an dem Unterschenkelteil 4 gelagert und somit Teil eines Prothesenkniegelenkes. Oberhalb des Prothesenfußes 3, also im Anschluss an das proximale Ende des Prothesenfußes 3, ist ein Prothesenelement 6 in Gestalt einer Knöchelmanschette angeordnet, die einen Grundkörper 101 aufweist. Die Knöchelmanschette 6 überdeckt den Übergangsbereich von dem Prothesenfuß 3 zum Unterschenkelteil 4 und erstreckt sich ungefähr über ein Drittel der Länge des Unterschenkelteils 4. Die Knöchelmanschette 6 umgibt den Unterschenkelteil 4 vollständig und ist an dem Prothesenfuß 3 über nicht dargestellte Formschlusselemente reversibel festgelegt.

In der Figur 9 ist die Protheseneinrichtung 2 mit der Knöchelmanschette 6 von schräg hinten gezeigt. In dem Unterschenkelteil 4 sind Dämpfereinrichtungen und eine Steuerelektronik sowie das Prothesenkniegelenk zu erkennen. Die Knöchelmanschette 6 selbst weist an der posterioren Seite ein an dem Grundkörper 101 angeordnetes Versteifungselement 100 auf, das an der posterioren Seite der Protheseneinrichtung 2, also im Bereich der Wade angeordnet ist und sich im Wesentlichen in Längsrichtung des Unterschenkelteils 4 erstreckt. Das Versteifungselement 100 dient einerseits zur Gewährleistung der Stabilität der Knöchelmanschette 6 in deren Längserstreckung und verhindert, dass während der Benutzung der Knöchelmanschette 6 diese von dem Unterschenkelteil 4 in Richtung auf den Prothesenfuß 3 herunterrutscht und gegebenenfalls Falten schlägt. Das Versteifungselement 100 dient anderseits zum Verbinden der einander gegenüberliegenden Enden oder lateraler Ränder des Zuschnitts 10, aus dem der Grundkörper 101 gebildet wird. Der Grundkörper 101 ist im dargestellten Ausführungsbeispiel nicht als kreisförmiger oder ovaler Materialzuschnitt mit einem geschlossenen Querschnitt, sondern als ein Flachzuschnitt mit einem offenen Querschnitt ausgebildet ist, wobei die einander gegenüberliegenden Kanten 11, 12 des Zuschnittes 10 über das Versteifungselement 100, das gleichzeitig die Funktion des Formschlusselementes 5 ausübt, miteinander verbunden werden, so dass ein Hohlraum zur Aufnahme der Protheseneinrichtung 2, insbesondere des unteren oder distalen Teils des Unterschenkelteils 4 bereitgestellt wird.

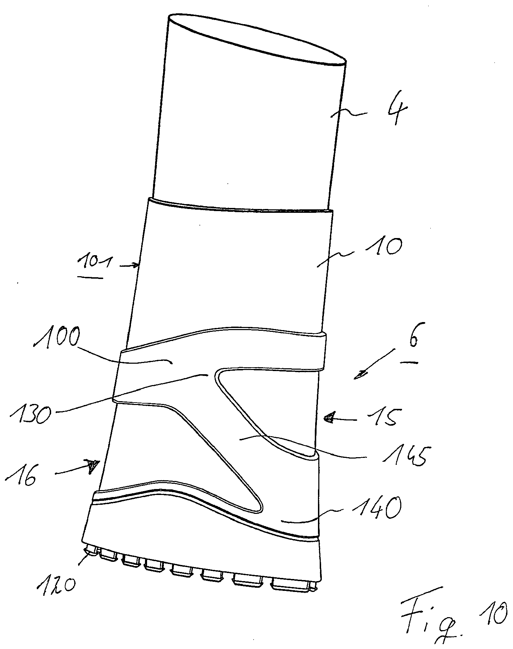

In der Figur 10 ist eine Variante des Prothesenelementes als Knöchelmanschette 6 gezeigt, die an einem Unterschenkelteil 4 angeordnet ist. Der Prothesenfuß ist in der Figur 10 nicht dargestellt. Die Knöchelmanschette 6 sieht einen Grundkörper 101 mit einem Zuschnitt 10 aus einem flexiblen Material, insbesondere einem Textil, einem Schaumstoff oder einer Kombination mehrerer flexibler Werkstoffe vor. Der Grundkörper 101 ist im dargestellten Ausführungsbeispiel schlauchartig ausgebildet und weist einen geschlossenen Querschnitt auf, der durch eine Verbindung einander gegenüberliegenden Ränder des Zuschnittes 10 hergestellt worden ist.. Auf der Außenseite des Grundkörpers 101 ist ein Versteifungselement 100 in Gestalt eines Rahmens angeordnet, der unter anderem einen umlaufenden, distalen Abschluss der Knöchelmanschette 6 ausbildet. An dem distalen Ende des Versteifungselementes 100 sind Befestigungselemente 120 zur formschlüssigen Festlegung an dem Prothesenfuß ausgeformt. Neben der dargestellten einstückigen Variante mit angeformten Befestigungselementen 120 ist es möglich, an dem Versteifungselement 100 separate Befestigungselemente festzulegen, um diese dann mit Formschlusselementen an dem Prothesenfuß in Eingriff zu bringen und dort eine formschlüssige Festlegung der Knöchelmanschette 6 an dem Prothesenfuß zu bewirken.

An dem distalen Ende des Grundkörpers 101 ist das Versteifungselement 100 als umlaufender geschlossener Ring ausgebildet, von dem sich von dem posterioren Ende, das in der Figur 10 rechts ausgebildet ist, ein schräg nach oben verlaufender Rahmenabschnitt 145 verläuft. Der Übergang von dem unteren, ringförmigen Abschluss zu dem nach oben zeigenden Rahmenabschnitt 145 ist als eine Scharniereinrichtung 140 ausgebildet, so dass eine federnde Bewegung des anterioren, oder vorderen Bereiches des Versteifungselementes 100 nach unten oder in distaler Richtung erfolgen kann. Dazu ist in dem Versteifungselement 100 eine anteriore Ausnehmung 16 vorgesehen, die von dem Material des Grundkörpers 101 ausgefüllt ist.

Von dem sich schräg nach vorn erstreckenden Rahmenabschnitt 145 des Versteifungselementes 100 erstreckt sich leicht nach oben geneigt ein oberer Rahmenabschnitt 135 schräg nach hinten und nach oben, also zu dem proximalen Ende der Knöchelmanschette 6 hin, und bildet so einen Bügel aus, der sich um den posterioren Teil des Grundkörpers 101 oberhalb des posterioren Scharniers 140 herum erstreckt. An dem Übergang von dem schräg nach vorne weisenden Rahmenabschnitt 145 zu dem schräg nach hinten weisenden Rahmenabschnitt 135 ist ein zweites Scharnierelement oder eine zweite Scharniereinrichtung 130 angeordnet und ausgebildet, so dass der sich schräg nach hinten erstreckende Rahmenabschnitt 135 nach unten verlagert werden kann. Unterhalb des oberen Rahmenabschnittes 135 ist eine posteriore Ausnehmung 15 ausgebildet, die ebenfalls von dem Material 1 des Grundkörpers 101 verschlossen ist. Wird eine Plantarflexion des Prothesenfußes 3 vorgenommen, ergibt sich analog zu einer natürlichen Fußbewegung eine Verlagerung des oberen, nach hinten weisenden Rahmenabschnittes 135 um die Scharniereinrichtung 130 und eine flexible Verformung des Materials 1 des Grundkörpers 101 innerhalb der Ausnehmung 15 durch eine Stauchung, während das flexible Material 1 des Grundkörpers 101 im Bereich der anterioren Ausnehmung 16 gestreckt wird und der sich nach vorne erstreckende Rahmenteil 145 nach oben verlagert wird. Bei einer Dorsalflexion erfolgt eine Stauchung im Bereich der anterioren Ausnehmung 16 und eine Streckung im Bereich der posterioren Ausnehmung 15. Neben einer einteiligen Ausgestaltung des Versteifungselementes 100 ist es vorgesehen, dass es mehrteilig oder modular aufgebaut ist, so dass beispielsweise der untere, ringförmige Rahmen mit den oberen Rahmenabschnitten verbunden ist, ggf. gelenkig über ein Scharnier oder mehrere Scharniere verbunden.

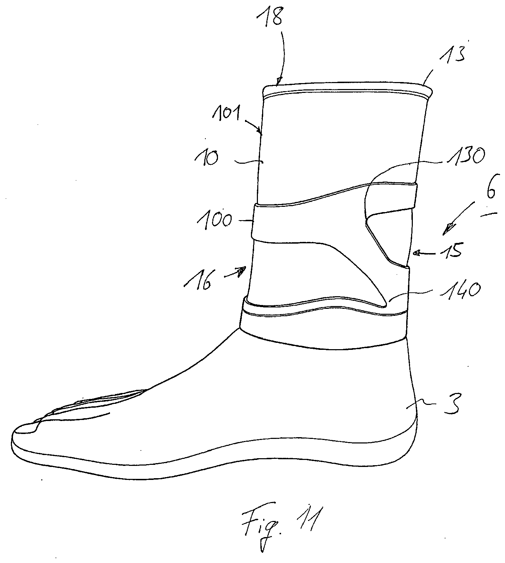

Figur 11 zeigt die Ausführungsform gemäß Figur 10 in einem an dem Prothesenfuß 3 montierten Zustand in Seitenansicht. Die Befestigungselemente 120 sind in nicht dargestellten Ausnehmungen im oberen Rand des Prothesenfußabschlusses eingeführt und ermöglichen es, eine bündige Montage der Knöchelmanschette 6 an dem Prothesenfuß 3 durchzuführen. Neben einer sicheren Festlegung gegen eine Auszugsbewegung, also von dem Prothesenfuß 3 weg, erfolgt über die Befestigungselemente 120 auch eine Anpassung der Knöchelmanschette 6 an die Kontur des jeweiligen Prothesenfußes 3, da neben dem Grundkörper 101 auch das Versteifungselement eine Flexibilität und Elastizität aufweist, so dass unterschiedliche Prothesenfüße 3 mit nur einer Knöchelmanschette 6 versorgt werden können. In der Figur 11 ist zu erkennen, dass das Versteifungselement 100 den Grundkörper 101 in der gewünschten Form hält, so dass der Grundkörper 101 einen Hohlraum umschließt, der über den proximalen Rand des Versteifungselementes 100 hinausragt. Auf der Innenseite am proximalen Rand 13 des Grundkörpers 101 kann eine reibungsvermindernde Beschichtung 18 angeordnet sein, um eine Relativbewegung zwischen dem nicht dargestellten Unterschenkelteil 4 und dem Material des Grundkörpers 101 zu erleichtern.

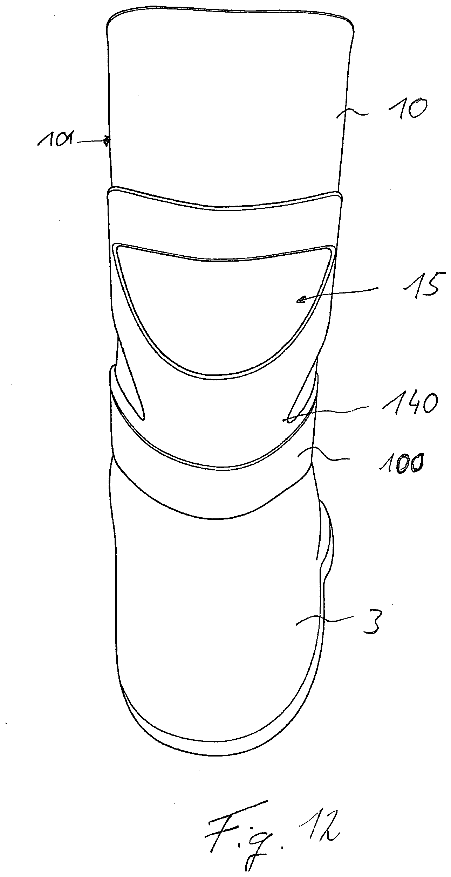

Figur 12 zeigt die Ausführungsform gemäß der Figur 11 in Rückansicht, es ist der Prothesenfuß 3 mit dem im Umfang bündig abschließenden, umlaufenden Versteifungselement 100 zu erkennen. Im posterioren Bereich, also im Bereich der Achillesferse, ist oberhalb des distalen Ringes des Versteifungselementes 100 die Scharniereinrichtung 140 ausgebildet, die durch die Ausnehmung 16 ausgebildet wird. Die Scharniereinrichtung 140 ermöglicht es, dass der sich nach oben, also zum proximalen Ende der Knöchelmanschette 6 erstreckende Teil oder Abschnitt des Versteifungselementes 100 leicht eingebeugt werden kann, bei dem posterioren Scharnier 140 bedeutet dies, dass die sich nach oben anschließenden Abschnitte des Versteifungselementes 100 bei einer Dorsalflexion nach vorne und unten verlagern können, also in Richtung auf den Prothesenfuß. Bei einer Plantarflexion übernimmt das obere bzw. anteriore Scharnier 130 diese Funktion und ermöglicht es dem posterioren, proximalen Abschnitt des Versteifungselementes 100 eine Verlagerung in Distalrichtung, so dass die posteriore Ausnehmung 15 verkleinert wird und das Material des Grundkörpers 101 komprimiert bzw. gestaucht wird.

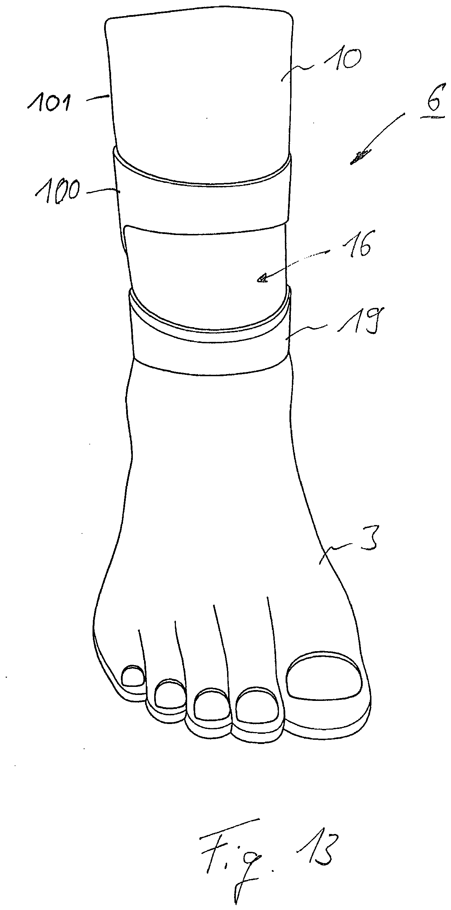

Figur 13 zeigt die Ausführungsform gemäß der Figuren 11 und 12 in einer Frontalansicht. Es ist zu erkennen, dass das Versteifungselement 100 im anterioren Bereich eine Ausnehmung 16 aufweist, die durch den Grundkörper 101 ausgefüllt ist. Das Versteifungselement 100 weist im distalen Bereich einen umlaufenden Ring 19 mit einem geschlossenen Querschnitt auf. Das Versteifungselement 100 dient zur Erhöhung der Eigenstabilität der Knöchelmanschette 6 und stellt gleichzeitig einen mechanischen Schutz gegen Umwelteinflüsse für das Knöchelgelenk oder die in dem Unterschenkelteil 4 angeordnete Elektronik bereit. Das Versteifungselement 100 kann auf dem Material 1 des Grundkörpers 101 aufgeschweißt, aufgeklebt, aufgenäht oder aufgespritzt sein. Im dargestellten Ausführungsbeispiel ist das Versteifungselement 100 auf der Außenseite des Grundkörpers 101 angeordnet, es besteht auch die Möglichkeit, dass es auf der Innenseite des Grundkörpers angeordnet ist oder den Grundkörper 101 einschließt, also auf der Außen- und Innenseite angeordnet ist.

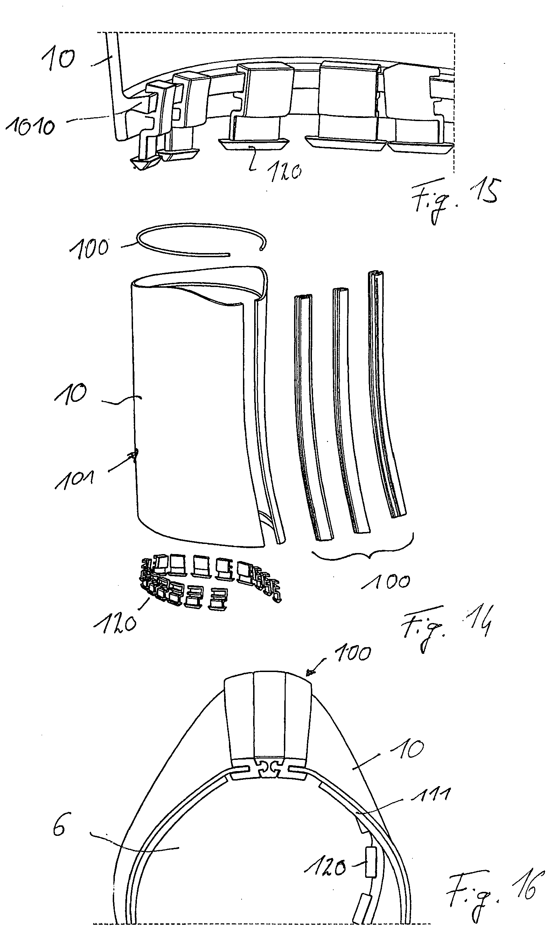

Eine Variante der Erfindung ist in der Figur 14 dargestellt, die eine Ausführungsform gemäß der Figur 8 eine Einzelteildarstellung zeigt. Der Grundkörper 101 ist als im Wesentlichen flächiger Zuschnitt 10 ausgestaltet und so geformt, dass die einander gegenüberliegenden Kanten zusammengefügt werden können, um einen schlauchartigen Hohlkörper auszubilden. Das Versteifungselement 100 ist im dargestellten Ausführungsbeispiel mehrteilig ausgebildet und sieht zwei Seitenschienen vor, die an dem Grundkörper 101 festgelegt werden. Diese Seitenschienen werden über eine Mittelschiene formschlüssig miteinander verbunden. Die Mittelschiene weist dazu Ausnehmungen auf, in die Formschlusselemente der Seitenschienen eingreifen. Um eine Umfangsstabilität zu erreichen, ist ein weiteres Versteifungselement 100 vorgesehen, das im dargestellten Ausführungsbeispiel am proximalen Ende des Grundkörpers 101 angeordnet ist. Das zweite Versteifungselement 100 kann als Federdraht oder Kunststoffspange ausgebildet sein.

An dem distalen Ende des Grundkörpers 101 sind separate Befestigungselemente 120 vorgesehen, die dort formschlüssig festgelegt sind und Vorsprünge aufweisen, mit denen die Befestigungselemente 120 und damit auch der Grundkörper 101 mit dem Versteifungselement 100 an dem Prothesenfuß festgelegt werden können.

Figur 15 zeigt eine vergrößerte Detailansicht die Innenseite des distalen Endes des Grundkörpers 101, an dem ein umlaufender Vorsprung 1010 angeordnet ist, der beispielsweise daran angespritzt oder daran ausgebildet sein kann. Der Vorsprung 1010 weist einen V-förmigen Querschnitt auf, der sich zur Außenwand des Grundkörpers 101 hin verjüngt. Die Befestigungselemente 120 weisen eine schwalbenschwanzartige, zur Form des Vorsprunges 1010 korrespondierende Ausnehmung auf, so dass sie formschlüssig in den Vorsprung 1010 eingreifen können. An dem distalen Ende der Befestigungselemente 120 sind pfeilartige Vorsprünge ausgebildet, um eine formschlüssige Verriegelung über das Ineingrifftreten mit korrespondierenden Ausnehmungen in dem Prothesenfuß zu ermöglichen. Die Befestigungselemente 120 sind reversibel und verschieblich an dem Grundkörper 101 festgelegt, so dass unterschiedliche Anzahlen von Befestigungselementen 120 an den jeweiligen Zuschnitten festgelegt werden können. Durch die Verschiebbarkeit der Befestigungselemente 120 an dem Grundkörper 101 ist es möglich, unterschiedliche Prothesenfußgrößen mit einem Zuschnitt des Grundkörpers 101 zu bedienen oder gegebenenfalls mit geringen Modifikationen eine Anpassung zu ermöglichen. Je größer die Prothesenfüße sind, desto größer ist der Abstand zwischen den einzelnen Ausnehmungen für die jeweiligen Befestigungselemente, so dass eine Anpassung ohne verschiebliche Befestigungselemente 120 nicht beliebig erfolgen kann.

Figur 16 zeigt die fertig gefügte Knöchelmanschette 6 mit dem Grundkörper 101, in dem in Umfangsrichtung wirkenden Versteifungselement 100 sowie dem in Längserstreckung wirkenden Versteifungselement 100 mit den zwei Außenschienen und der formschlüssig daran festgelegten Mittelschiene. Durch die Versteifungselemente 100 wird ein geschlossener Hohlraum 6 ausgebildet, der die jeweilige Komponente der Protheseneinrichtung aufnimmt.

Neben der Anwendung als Knöchelmanschette kann das Prothesenelement 6 auch an anderen Prothesenkomponenten festgelegt werden und neben Abdeckungsfunktionen auch tragende und strukurelle Aufgaben übernehmen.

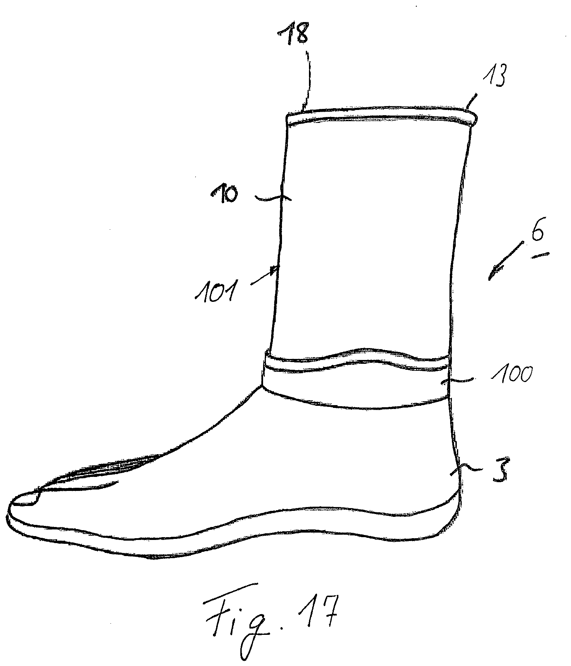

In der Figur 17 ist eine weitere Variante der Erfindung dargestellt. Die Variante entspricht im Wesentlichen der Ausführungsform gemäß der Figur 11, allerdings ist das Versteifungselement 100 im distalen Bereich des Grundkörpers 101 angeordnet oder ausgebildet, beispielsweise durch Aufspritzen, Anformen oder Ankleben einer separaten Materialschicht oder durch Aufdickung und gegebenenfalls Eigenschaftsveränderung des Grundmaterials 1, um eine erhöhte Formstabilität im distalen Bereich der Knöchelmanschette 6 zu erreichen. Der proximale Abschnitt des Grundkörpers 101, also derjenige Abschnitt, der proximal zum Versteifungselement 100 angeordnet ist, weist eine ausreichende Eigenstabilität auf, um den Zwischenraum zwischen dem Prothesenfuß 3 und dem nicht dargestellten Unterschenkelteil 4 zu überbrücken. Die Eigenschaftsveränderung des Materials 1 des Grundkörpers 101 bei einer Aufdickung kann durch Kompression oder thermische Behandlung erfolgen. Grundsätzlich ist es auch bei den anderen Ausführungsformen möglich, neben einer separaten Ausgestaltung des Versteifungselementes 100 diese oder dieses als Aufdickung oder Aufdickungen auszugestalten.

## Patentansprüche

1. Prothesenelement zur Verkleidung einer Prothese oder Ausbildung einer Prothesenkomponente, bestehend aus zumindest einem Zuschnitt (10) aus einem flächigen Grundmaterial (1), der unter Ausbildung eines Hohlkörpers gebogen ist, wobei im gebogenen Zustand einander gegenüberliegende Ränder (11, 12) miteinander verbunden sind,wobei an dem Grundmaterial (1) zumindest ein Versteifungselement (100) zur Erhöhung der Eigenstabilität des Prothesenelementes (6) angeordnet ist **dadurch gekennzeichnet, dass** an dem Zuschnitt (10) oder den Zuschnitten (10) Formschlusselemente (5), über die die Ränder (11, 12) aneinander festlegbar sind, oder Befestigungselemente (120) zur formschlüssigen Festlegung des Prothesenelementes (6) an einer Prothesenkomponente (3), an dem Grundmaterial (1) angeordnet sind.

2. Prothesenelement nach Anspruch 1, **dadurch gekennzeichnet, dass** auf dem Grundmaterial (1) mehrere Schnittlinien (21, 22, 23) für Zuschnitte (10) unterschiedlicher Größen aufgebracht sind.

3. Prothesenelement nach einem der voranstehenden Ansprüche, **dadurch gekennzeichnet, dass** es als Prothesenkosmetik oder als Last tragendes Strukturbauteil ausgebildet ist.

4. Prothesenelement nach einem der voranstehenden Ansprüche, **dadurch gekennzeichnet, dass** das Versteifungselement (100) aus einem Material mit einem gegenüber dem Grundmaterial (1) größeren Verformungswiderstand besteht.

5. Prothesenelement nach einem der voranstehenden Ansprüche, **dadurch gekennzeichnet, dass** das Versteifungselement (100) als Rahmen ausgebildet ist, der einen aus dem Grundmaterial (1) gebildeten Grundkörper (101) umgibt.

6. Prothesenelement nach einem der voranstehenden Ansprüche, **dadurch gekennzeichnet, dass** in dem Versteifungselement (100) zumindest eine Scharnierenrichtung (130, 140) zur Erleichterung einer Flexion um ein Gelenk angeordnet ist.

7. Prothesenelement nach einem der voranstehenden Ansprüche, **dadurch gekennzeichnet, dass** es medial-lateral symmetrisch ausgebildet ist.

8. Prothesenelement nach einem der voranstehenden Ansprüche, **dadurch gekennzeichnet, dass** das Versteifungselement (100) den distalen und/oder proximalen Abschluss des Prothesenelementes (6) bildet.

9. Prothesenelement nach einem der voranstehenden Ansprüche, **dadurch gekennzeichnet, dass** das Versteifungselement (100) Ausschnitte (15, 16) aufweist.

10. Prothesenelement nach einem der voranstehenden Ansprüche, **dadurch gekennzeichnet, dass** das Versteifungselement (100) den Querschnitt des Zuschnittes (10) schließt.

11. Prothesenelement nach einem der voranstehenden Ansprüche, **dadurch gekennzeichnet, dass** Befestigungselemente (120) zur formschlüssigen Festlegung des Prothesenelementes (6) an einem Prothesenfuß (3) an dem Versteifungselement (100) angeordnet sind.

12. Prothesenelement nach einem der voranstehenden Ansprüche, **dadurch gekennzeichnet, dass** an der proximalen Innenseite des Grundmaterials (1) eine reibungsvermindernde Beschichtung (18) angeordnet ist.

13. Verfahren zum Herstellen eines Prothesenelementes aus einem flächigen Grundmaterial (1), mit den Schritten:
a. Ausschneiden zumindest eines Zuschnittes (10) aus dem flächigen Grundmaterial (1)
b. Biegen des Zuschnittes (10) unter Ausbildung eines Hohlkörpers
c. Verbinden zweier einander nach dem Biegen gegenüberliegenden Ränder (11, 12) des Zuschnittes (10) über Formschlusselemente (5), die an dem Zuschnitt (10) angeordnet sind,
d. an dem Grundmaterial (1) wird zumindest ein Versteifungselement (100) zur Erhöhung der Eigenstabilität des Prothesenelementes (6) angeordnet.

14. Verfahren nach Anspruch 13, **dadurch gekennzeichnet, dass** der Zuschnitt (10) um die Prothese herum gebogen und anschließend die Ränder (11, 12) miteinander verbunden werden.

15. Verfahren nach Anspruch 13 oder 14, **dadurch gekennzeichnet, dass** der Zuschnitt (10) entlang vorher auf dem Grundmaterial (1) aufgebrachter Schnittlinien (21, 22, 23) ausgeschnitten wird.

16. Verfahren nach einem der Ansprüche 13 bis 15, **dadurch gekennzeichnet, dass** mehrere Zuschnitte (10) zur Ausbildung des Hohlkörpers miteinander verbunden werden.

## Claims

1. Prosthesis element for lining a prosthesis or forming a prosthesis component, consisting of at least one blank (10) of a flat base material (1), which is bent to form a hollow body, opposing edges (11, 12) in the bent state being connected to one another, wherein at least one stiffening element (100) is arranged on the base material (1) for increasing the inherent stability of the prosthesis element (6), **characterised in that** form-fitting elements (5) are arranged on the blank (10) or the blanks (10), by way of which the respective edges (11, 12) can be secured to one another, or fastening elements (120), for form-fitting securement of the prosthesis element (6) on a prosthesis component (3), are arranged on the base material (1).

2. Prosthesis element as claimed in claim 1, **characterised in that** a number of cutting lines (21, 22, 23) for blanks (10) of different sizes are provided on the base material (1).

3. Prosthesis element as claimed in one of the preceding claims, **characterised in that** it is formed as a cosmetic prosthesis cover or as a load-bearing structural component.

4. Prosthesis element as claimed in one of the preceding claims, **characterised in that** the stiffening element (100) consists of a material that has a greater deformation resistance than the base material (1).

5. Prosthesis element as claimed in one of the preceding claims, **characterised in that** the stiffening element (100) is formed as a frame which surrounds a main body (101) formed from the base material (1).

6. Prosthesis element as claimed in one of the preceding claims, **characterised in that** at least one hinge device (130, 140) is arranged in the stiffening element (100) to facilitate flexion about a joint.

7. Prosthesis element as claimed in one of the preceding claims, **characterised in that** it is symmetrically formed medial-laterally.

8. Prosthesis element as claimed in one of the preceding claims, **characterised in that** the stiffening element (100) forms the distal and/or proximal end of the prosthesis element (6).

9. Prosthesis element as claimed in one of the preceding claims, **characterised in that** the stiffening element (100) has cutouts (15, 16).

10. Prosthesis element as claimed in one of the preceding claims, **characterised in that** the stiffening element (100) closes the cross section of the blank (10).

11. Prosthesis element as claimed in one of the preceding claims, **characterised in that** fastening elements (120), for the form-fitting securement of the prosthesis element (6) on a prosthetic foot (3), are arranged on the stiffening element (100).

12. Prosthesis element as claimed in one of the preceding claims, **characterised in that** a friction-reducing coating (18) is arranged on the proximal inner side of the base material (1).

13. Method for producing a prosthesis element from a flat base material (1), with the steps of:
a. cutting out at least one blank (10) from the flat base material (1)
b. bending the blank (10) to form a hollow body
c. connecting two edges (11, 12) of the blank (10) that lie opposite one another after the bending via form-fitting elements (5), which are arranged on the blank (10),
d. at least one stiffening element (100) is arranged on the base material (1) for increasing the inherent stability of the prosthesis element (6).

14. Method as claimed in claim 13, **characterised in that** the blank (10) is bent around the prosthesis and the edges (11, 12) are subsequently connected to one another.

15. Method as claimed in claim 13 or 14, **characterised in that** the blank (10) is cut out along cutting lines (21, 22, 23) previously provided on the base material (1).

16. Method as claimed in one of claims 13 to 15, **characterised in that** a number of blanks (10) are connected to one another to form the hollow body.

## Revendications

1. Élément de prothèse pour habiller une prothèse ou pour réaliser un composant de prothèse, constitué par au moins un flan (10) d'un matériau de base surfacique (1), ledit flan étant cintré en formant un corps creux, et dans l'état cintré des bords (11, 12) mutuellement opposés sont reliés l'un à l'autre,
dans lequel
un élément de rigidification (100) destiné à augmenter la stabilité propre de l'élément de prothèse (6) est agencé sur le matériau de base (1),
**caractérisé en ce que**
sur le flan (10) ou sur les flans (10), des éléments à coopération de formes (5) permettant d'immobiliser les bords (11, 12) l'un sur l'autre ou des éléments de fixation (120) destinés à l'immobilisation par coopération de formes de l'élément de prothèse (6) sur un composant de prothèse (3) sont agencés sur le matériau de base (1).

2. Élément de prothèse selon la revendication 1,
**caractérisé en ce que**
plusieurs lignes de coupe (21, 22, 23) pour des flans (10) de différentes tailles sont appliquées sur le matériau de base (1).

3. Élément de prothèse selon l'une des revendications précédentes,
**caractérisé en ce que**
il est réalisé sous forme d'enjoliveur de prothèse ou sous forme de composant structurel portant une charge.

4. Élément de prothèse selon l'une des revendications précédentes,
**caractérisé en ce que**
l'élément de rigidification (100) est constitué en un matériau présentant une résistance à la déformation qui est supérieure à celle du matériau de base (1).

5. Élément de prothèse selon l'une des revendications précédentes,
**caractérisé en ce que**
l'élément de rigidification (100) est réalisé sous forme de cadre qui entoure un corps de base (101) formé par le matériau de base (1).

6. Élément de prothèse selon l'une des revendications précédentes,
**caractérisé en ce que**
au moins un moyen formant charnière (130, 140) pour faciliter une flexion autour d'une articulation est agencé dans l'élément de rigidification (100).

7. Élément de prothèse selon l'une des revendications précédentes,
**caractérisé en ce que**
il est réalisé avec symétrie médiale-latérale.

8. Élément de prothèse selon l'une des revendications précédentes,
**caractérisé en ce que**
l'élément de rigidification (100) constitue la terminaison distale et/ou proximale de l'élément de prothèse (6).

9. Élément de prothèse selon l'une des revendications précédentes,
**caractérisé en ce que**
l'élément de rigidification (100) présente des découpes (15, 16).

10. Élément de prothèse selon l'une des revendications précédentes,
**caractérisé en ce que**
l'élément de rigidification (100) referme la section transversale du flan (10).

11. Élément de prothèse selon l'une des revendications précédentes,
**caractérisé en ce que**
les éléments de fixation (120) pour immobiliser l'élément de prothèse (6) par coopération de formes sur un pied de prothèse (3) sont agencés sur l'élément de rigidification (100).

12. Élément de prothèse selon l'une des revendications précédentes,
**caractérisé en ce que**
un revêtement (18) réduisant la friction est agencé sur le côté intérieur proximal du matériau de base (1).

13. Procédé pour fabriquer un élément de prothèse à partir d'un matériau de base surfacique (1), comprenant les étapes consistant à :
a. découper au moins un flan (10) dans le matériau de base surfacique (1),
b. cintrer le flan (10) en formant un corps creux,
c. relier deux bords (11, 12) du flan (10), mutuellement opposés après le cintrage, par des éléments à coopération de formes (5) qui sont agencés sur le flan (10),
d. agencer au moins un élément de rigidification (100) sur le matériau de base (1) pour augmenter la stabilité propre de l'élément de prothèse (6).

14. Procédé selon la revendication 13,
**caractérisé en ce que**
le flan (10) est cintré autour de la prothèse et ensuite les bords (11, 12) sont reliés l'un à l'autre.

15. Procédé selon la revendication 13 ou 14,
**caractérisé en ce que**
le flan (10) est découpé le long de lignes de coupe (21, 22, 23) appliquées préalablement sur le matériau de base (1).

16. Procédé selon l'une des revendications 13 à 15,
**caractérisé en ce que**
plusieurs flans (10) sont reliés les uns aux autres pour former le corps creux.
